(19) Europäisches Patentamt
European Patent Office
Office européen des brevets

(11) Veröffentlichungsnummer: **0 206 141 A1**

(12) **EUROPÄISCHE PATENTANMELDUNG**

(21) Anmeldenummer: **86108020.8**

(51) Int. Cl.4: **C07C 127/22**, A01N 47/34

(22) Anmeldetag: **12.06.86**

(30) Priorität: **15.06.85 DE 3521557**

(43) Veröffentlichungstag der Anmeldung:
**30.12.86 Patentblatt 86/52**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Lange, Arno, Dr.**
**Oberes Geistal 3 b**
**D-6702 Bad Dürkheim(DE)**
Erfinder: **Neubauer, Hans-Jürgen, Dr.**
**B 4,2**
**D-6800 Mannheim 1(DE)**
Erfinder: **Adolphi, Heinrich, Dr.**
**Kalmitweg 11**
**D-6703 Limburgerhof(DE)**

(54) **N-Benzoyl-N'-p-aminophenylharnstoffe, ihre Herstellung und Verwendung zur Bekämpfung von Insekten und Akariden.**

(57) N-Benzoyl, N'-(aminophenyl)harnstoffe der Formel

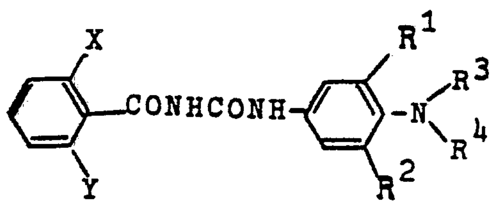

(I),

in der X für F, Cl oder H,

Y für F, Cl,

$R^1$ und $R^2$ (die gleich oder verschieden sein können) für Cl, Br,

$R^3$ für einen gesättigten, olefinisch oder acetylenisch ungesättigten Kohlenwasserstoffrest mit 1 bis 5 Kohlenstoffatomen steht, der einen Phenylrest trägt und $R^4$ für H oder einen acyclischen Kohlenwasserstoffrest mit 1 bis 5 Kohlenstoffatomen oder die Bedeutung von $R^3$ steht, wobei $R^3$ und $R^4$ gleich oder verschieden sein können, deren Herstellung und Verwendung als Schädlingsbekämpfungsmittel.

EP 0 206 141 A1

**N-Benzoyl-N'-p-aminophenylharnstoffe, ihre Herstellung und Verwendung zur Bekämpfung von Insekten und Akariden**

Es ist bekannt, daß N-Benzoyl-Nx-phenylharnstoffe insektizid wirksam sind (DE-OS 2 123 236, DE-OS 2 926 480 (= US 4 350 706), DE-OS 3 132 020). Aus der zuletzt genannten Druckschrift sind insbesondere N-Benzoyl-N'-(p-aminophenyl)-harnstoffe bekannt, deren p-Aminogruppe durch Alkyl bzw. Alkylen oder Phenyl substituiert sein kann.

Diese Verbindungen weisen jedoch entweder eine zu geringe spezifische Wirkung auf oder sind nicht einfach genug herzustellen.

Die Erfindung löst die Aufgabe, neuartige einfach herstellbare, hochwirksame N-Benzoyl-N'-(p-aminophenyl)-harnstoffe zu finden.

Es wurde gefunden, daß N-Benzoyl-N'-phenylharnstoffe der Formel

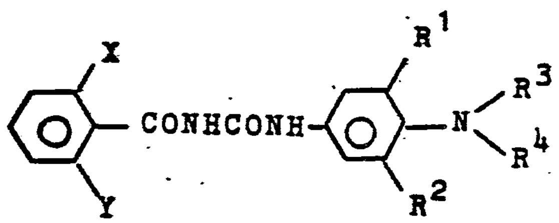

(I),

in der

X für F, Cl, oder H,

Y für F, Cl,

$R^1$ und $R^2$ (die gleich oder verschieden sein können) für Cl, Br,

$R^3$ für einen gesättigten, olefinisch oder acetylenisch ungesättigten Kohlenwasserstoffrest mit 1 bis 5 Kohlenstoffatomen steht, der einen Phenylrest trägt, und

$R^4$ für H oder einen acyclischen Kohlenwasserstoffrest mit 1 bis 5 Kohlenstoffatomen oder die Bedeutung von $R^3$ steht, wobei $R^3$ und $R^4$ gleich oder verschieden sein können, insektizid sehr wirksam sind.

Sie sind bekannten Benzoylharnstoffen ähnlicher Struktur in ihrer Wirkung überlegen. Beispiele für phenylsubstituierte Reste $R^3$ sind: Benzyl, Phenethyl, 3-Phenylpropyl-(1), 4-Phenylbutyl-(1), 10-Phenyldecyl-(1), Phenethenyl, Phenethinyl, 2-Chlorbenzyl, 4-Chlorbenzyl, 5-(4-Methylphenyl)-pentyl-(1).

$R^4$ kann z.B. primäres, sekundäres oder tertiäres Alkyl mit 1 bis 10 Kohlenstoffatomen, wie Methyl, Ethyl, n-Propyl, Isopropyl, sec.-Butyl, Pentyl-(3), 2,4-Dimethyl-pentyl-(3), n-Heptyl, 2,6-Dimethylheptyl-(4), Methyl, Ethyl, n-Propyl, n-Butyl, n-Pentyl, n-Hexyl, n-Dodecyl, 6,6,4-Trimethylhexyl-(1), t-Butyl, 3-Ethylpentyl-(3)-, 2,2,5-Trimethylpentyl-(1) oder olefinisch bzw. acetylenisch ungesättigt wie Allyl, Methalkyl oder Propargyl sein.

Besonders wirtschaftlich herstellbar sind Verbindungen, bei denen $R^1$ = $R^2$ ist und Cl oder Br bedeutet. Bevorzugte N-Benzoyl-N'-phenylharnstoffe sind Verbindungen der Formel I, in der X Fluor oder Chlor und Y Fluor oder Wasserstoff bedeuten, besonders bevorzugt sind Verbindungen mit X = Y = Fluor. $R^3$ ist bevorzugt Benzyl, Phenethyl oder 1-Phenylpropyl-(1).

$R^4$ ist vorzugsweise ein n-Alkylrest mit bis zu 4 Kohlenstoffatomen; besonders bevorzugt sind Methyl u. Ethyl. Man erhält die N-Benzoyl-N'-phenylharnstoffe der Formel I z.B. durch Umsetzen von entsprechenden Benzoylisocyanaten mit entsprechenden Aminoanilinen oder von Benzoylamiden mit Aminophenylisocyanaten in Gegenwart eines inerten organischen Lösungsmittels bei einer Temperatur von i.a. unter 80 °C. Hinweise zum Ablauf der Umsetzungen können u.a. den obengenannten Druckschriften entnommen und auf die erfindungsgemäßen Stoffe leicht angewandt werden. Die für die Herstellung erforderlichen Vorprodukte sind entweder bekannt -dies trifft insbesondere für die benötigten substituierten Benzoylisocyanate zu- oder sie können mit dem fachmännischen Allgemeinwissen aus z.B. p-Phenylendiamin oder -(substituierten) Nitranilinen hergestellt werden. Hinweise können zusätzlich entnommen werden aus J. Org. 28, 1805 -1811 (1963); Weygand-Hilgetag: Organisch-chemische Experimentierkunst, 4.Auflage, 1970, S. 475, 576, J. A. Barth Verlag Leipzig.

Die erfindungsgemäßen Stoffe sind bei Raumtemperatur durchweg feste Stoffe, die, falls sie nicht schon in reiner Form anfallen, z.B. durch Umkristallisieren gereinigt werden können. Zu ihrer Charakterisierung eignen sich Elementaranalyse und Schmelzpunkt sowie ggf. auch spektroskopische Methoden.

Beispiele für die Herstellung von Vorprodukten

A) 1,17 Mol 3,5-Dichlor, 4-bromnitrobenzol und 2,34 Mol N-Methylbenzylamin werden während 4 h bei 85 °C in 300 ml Dimethylsulfoxid umgesetzt, in verdünnte Salzsäure eingetragen, mit Ether aufgenommen, mit Wasser gewaschen, getrocknet und i./V. eingeengt. Man erhält 349 g N-Benzyl, N-methyl, 2,6-dichlor, 4-nitranilin als Öl, das dünnschichtchromatographisch einheitlich ist. Die für charakteristischen IR-Absorptionslinien werden bei 1526 und 1336 $cm^{-1}$ gefunden.

B) 21,5 g das nach Vorschrift (A) erhaltenen Anilins werden in 150 ml Tetrahydrofuran gelöst und mit 8 g Raney-Nickel (mit wenig Pt-IV-chlorid aktiviert) während 42 h bei 50 °C und 200 Bar hydriert. Man filtriert ab, und engt ein und erhält 16,9 g N-Benzyl, N-methyl, 2,6-Dichlor, 4-aminoanilin; charakteristische IR-Linien: 3390 und 3480 $cm^{-1}$ ($NH_2$).

Beispiel für die Herstellung eines erfindungsgemäßen Stoffes (I)

5 g Phenylendiamin (B) werden in 50 ml Toluol gelöst und bei Raumtemperatur tropfenweise mit 3,7 g 2,6-Difluorbenzoylisocyanat in 5 ml Toluol versetzt. Es wird über Nacht nachgerührt, abgesaugt und getrocknet und erhält 5 g der Verbindung, die in der nachstehenden Tabelle unter Nr. 1 aufgeführt ist.

Es wurden folgende Harnstoffe hergestellt - (soweit deren Schmelzpunkte (Fp) angegeben sind):

| Nr. | X | Y | $R^1$ | $R^2$ | $R^3$ | $R^4$ | Fp.: |
|---|---|---|---|---|---|---|---|
| 1 | F | F | Cl | Cl | $-CH_2-C_6H_5$ | $CH_3$ | 177 - 182° |
| 2 | F | F | Cl | Cl | $-C_2H_4-C_6H_5$ | $CH_3$ | |
| 3 | F | F | Cl | Cl | $n-C_3H_6-C_6H_5$ | $CH_3$ | 143 - 145° |
| 4 | F | F | Cl | Cl | $n-C_4H_8-C_6H_5$ | $CH_3$ | |
| 5 | F | F | Cl | Cl | $-CH_2-C_6H_5$ | $C_2H_5$ | 136 - 138° |
| 6 | F | F | Cl | Cl | $-CH_2-C_6H_5$ | $i-C_3H_7$ | |
| 7 | F | F | Cl | Cl | $-CH_2-C_6H_5$ | $t-C_4H_9$ | |
| 8 | F | F | Cl | Cl | $-CH_2C_6H_5$ | $-CH_2-C{\equiv}CH$ | |
| 9 | F | F | Cl | Cl | $-CH_2-C_6H_5$ | $-CH_2-C_6H_5$ | |
| 10 | F | F | Br | Br | $-CH_2-C_6H_5$ | $CH_3$ | 163 - 165° |
| 11 | F | F | Br | Br | $-C_2H_4-C_6H_5$ | $CH_3$ | |
| 12 | F | F | Br | Br | $n-C_3H_6-C_6H_5$ | $CH_3$ | |
| 13 | F | F | Br | Br | $n-C_4H_8-C_6H_5$ | $CH_3$ | |
| 14 | F | F | Br | Br | $-CH_2-C_6H_5$ | $C_2H_5$ | |
| 15 | F | F | Br | Br | $-CH_2-C_6H_5$ | $i-C_3H_7$ | |
| 16 | F | F | Br | Br | $-CH_2-C_6H_5$ | $t-C_4H_9$ | |
| 17 | F | F | Br | Br | $-CH_2-C_6H_5$ | $-CH_2-C{\equiv}CH$ | |
| 18 | F | F | Br | Br | $-CH_2-C_6H_5$ | $-CH_2C_6H_5$ | |
| 19 | Cl | Cl | Cl | Cl | $-CH_2-C_6H_5$ | $CH_3$ | 112 - 115° |
| 20 | Cl | Cl | Cl | Cl | $-C_2H_4-C_6H_5$ | $CH_3$ | |
| 21 | Cl | Cl | Cl | Cl | $n-C_3H_6-C_6H_5$ | $CH_3$ | |
| 22 | Cl | Cl | Cl | Cl | $n-C_4H_8-C_6H_5$ | $CH_3$ | |
| 23 | Cl | Cl | Cl | Cl | $-CH_2-C_6H_5$ | $C_2H_5$ | |
| 24 | Cl | Cl | Cl | Cl | $-CH_2-C_6H_5$ | $i-C_3H_7$ | |

| Nr. | X | Y | $R^1$ | $R^2$ | $R^3$ | $R^4$ | Fp.: |
|---|---|---|---|---|---|---|---|
| 25 | Cl | Cl | Cl | Cl | $-CH_2-C_6H_5$ | $t-C_4H_9$ | |
| 26 | Cl | Cl | Cl | Cl | $-CH_2-C_6H_5$ | $-CH_2-C{\equiv}CH$ | |
| 27 | Cl | Cl | Cl | Cl | $-CH_2-C_6H_5$ | $-CH_2-C_6H_5$ | |
| 28 | Cl | Cl | Br | Br | $-CH_2C_6H_5$ | $CH_3$ | |
| 29 | Cl | Cl | Br | Br | $-C_2H_4-C_6H_5$ | $CH_3$ | |
| 30 | Cl | Cl | Br | Br | $n-C_3H_6-C_6H_5$ | $CH_3$ | |
| 31 | Cl | Cl | Br | Br | $n-C_4H_8C_6H_5$ | $CH_3$ | |
| 32 | Cl | Cl | Br | Br | $-CH_2-C_6H_5$ | $C_2H_5$ | |
| 33 | Cl | Cl | Br | Br | $-CH_2-C_6H_5$ | $i-C_3H_7$ | |
| 34 | Cl | Cl | Br | Br | $-CH_2-C_6H_5$ | $t-C_4H_9$ | |
| 35 | Cl | Cl | Br | Br | $-CH_2-C_6H_5$ | $-CH_2-C{\equiv}CH$ | |
| 36 | Cl | Cl | Br | Br | $-CH_2-C_6H_5$ | $-CH_2-C_6H_5$ | |
| 37 | H | Cl | Cl | Cl | $-CH_2-C_6H_5$ | $CH_3$ | |
| 38 | H | Cl | Cl | Cl | $-C_2H_4-C_6H_5$ | $CH_3$ | |
| 39 | H | Cl | Cl | Cl | $n-C_3H_6-C_6H_5$ | $CH_3$ | 132 - 134° |
| 40 | H | Cl | Cl | Cl | $n-C_4H_8-C_6H_5$ | $CH_3$ | |
| 41 | H | Cl | Cl | Cl | $-CH_2-C_6H_5$ | $C_2H_5$ | 134 - 136° |
| 42 | H | Cl | Cl | Cl | $-CH_2-C_6H_5$ | $i-C_3H_7$ | |
| 43 | H | Cl | Cl | Cl | $-CH_2-C_6H_5$ | $t-C_4H_9$ | |
| 44 | H | Cl | Cl | Cl | $-CH_2-C_6H_5$ | $-CH_2-C{\equiv}CH$ | |
| 45 | H | Cl | Cl | Cl | $-CH_2-C_6H_5$ | $-CH_2-C_6H_5$ | |
| 46 | H | Cl | Br | Br | $-CH_2-C_6H_5$ | $CH_3$ | 156 - 159° |
| 47 | H | Cl | Br | Br | $-C_2H_4-C_6H_5$ | $CH_3$ | |
| 48 | H | Cl | Br | Br | $n-C_3H_6-C_6H_5$ | $CH_3$ | |
| 49 | H | Cl | Br | Br | $n-C_4H_8-C_6H_5$ | $CH_3$ | |
| 50 | H | Cl | Br | Br | $-CH_2-C_6H_5$ | $C_2H_5$ | |
| 51 | H | Cl | Br | Br | $-CH_2-C_6H_5$ | $i-C_3H_7$ | |
| 52 | H | Cl | Br | Br | $-CH_2C_6H_5$ | $t-C_4H_9$ | |
| 53 | H | Cl | Br | Br | $-CH_2-C_6H_5$ | $-CH_2-C{\equiv}CH$ | |
| 54 | H | Cl | Br | Br | $-CH_2H_6H_5$ | $-CH_2-C_6H_5$ | |

| Nr. | X | Y | $R^1$ | $R^2$ | $R^3$ | $R^4$ | Fp.: |
|---|---|---|---|---|---|---|---|
| 55 | H | Br | Cl | Cl | $-CH_2-C_6H_5$ | $CH_3$ | |
| 56 | F | Cl | Cl | Cl | $-C_2H_4-C_6H_5$ | $CH_3$ | |
| 57 | F | Cl | Cl | Cl | $n-C_3H_6-C_6H_5$ | $CH_3$ | |
| 58 | F | Cl | Cl | Cl | $n-C_4H_8-C_6H_5$ | $CH_3$ | |
| 59 | F | Cl | Cl | Cl | $-CH_2-C_6H_5$ | $C_2H_5$ | |
| 60 | F | Cl | Cl | Cl | $-CH_2-C_6H_5$ | $i-C_3H_7$ | |
| 61 | F | Cl | Cl | Cl | $-CH_2-C_6H_5$ | $t-C_4H_9$ | |
| 62 | F | Cl | Cl | Cl | $-CH_2-C_6H_5$ | $-CH_2-C\equiv CH$ | |
| 63 | F | Cl | Cl | Cl | $-CH_2-C_6H_5$ | $-CH_2-C_6H_5$ | |
| 64 | H | $CH_3$ | Cl | Cl | $-CH_2-C_6C_5$ | $CH_3$ | |
| 65 | F | F | Br | Cl | $-C_2H_4-C_6H_5$ | $CH_3$ | |
| 66 | F | F | Br | Cl | $n-C_3H_6-C_6H_5$ | $CH_3$ | |
| 67 | F | F | Br | Cl | $n-C_4H_8-C_6H_5$ | $CH_3$ | |
| 68 | F | F | Br | Cl | $-CH_2-C_6H_5$ | $C_2H_5$ | |
| 69 | F | F | Br | Cl | $-CH_2-C_6H_5$ | $i-C_3H_7$ | |
| 70 | F | F | Br | Cl | $-CH_2-C_6H_5$ | $t-C_4H_9$ | |
| 71 | F | F | Br | Cl | $-CH_2-C_6H_5$ | $-CH_2-C\equiv CH$ | |
| 72 | F | F | Br | Cl | $-CH_2-C_6H_5$ | $-CH_2-C_6H_5$ | |
| 73 | F | F | Br | Cl | $-CH_2-C_6H_5$ | $CH_3$ | 154 - 168° |
| 74 | H | Cl | Br | Cl | $-C_2H_4-C_6H_5$ | $CH_3$ | |
| 75 | H | Cl | Br | Cl | $n-C_3H_6-C_6H_5$ | $CH_3$ | |
| 76 | H | Cl | Br | Cl | $n-C_4H_8-C_6H_5$ | $CH_3$ | |
| 77 | H | Cl | Br | Cl | $-CH_2-C_6H_5$ | $C_2H_5$ | |
| 78 | H | Cl | Br | Cl | $-CH_2-C_6H_5$ | $i-C_3H_7$ | |
| 79 | H | Cl | Br | Cl | $-CH_2-C_6H_5$ | $t-C_4H_9$ | |
| 80 | H | Cl | Br | Cl | $-CH_2-C_6H_5$ | $-CH_2-C\equiv CH$ | |
| 81 | H | Cl | Br | Cl | $-CH_2-C_6H_5$ | $-CH_2-C_6H_5$ | |
| 82 | H | Cl | Br | Cl | $-CH_2-C_6H_5$ | $CH_3$ | |
| 83 | Cl | Cl | Br | Cl | $-C_2H_4-C_6H_5$ | $CH_3$ | 157 - 187° |
| 84 | Cl | Cl | Br | Cl | $n-C_3H_6-C_6H_5$ | $CH_3$ | |

| Nr. | X | Y | $R^1$ | $R^2$ | $R^3$ | $R^4$ | Fp.: |
|---|---|---|---|---|---|---|---|
| 85 | Cl | Cl | Br | Cl | $n-C_4H_9-C_6H_5$ | $CH_3$ | |
| 86 | Cl | Cl | Br | Cl | $-CH_2-C_6H_5$ | $C_2H_5$ | |
| 87 | Cl | Cl | Br | Cl | $-CH_2-C_6H_5$ | $i-C_3H_7$ | |
| 88 | Cl | Cl | Br | Cl | $-CH_2-C_6H_5$ | $t-C_4H_9$ | |
| 89 | Cl | Cl | Br | Cl | $-CH_2-C_6H_5$ | $-CH_2-C=CH$ | |
| 90 | Cl | Cl | Br | Cl | $-CH_2-C_6H_5$ | $-CH_2-C_6H_5$ | |

Als Vergleichsmittel wurde eingesetzt

I 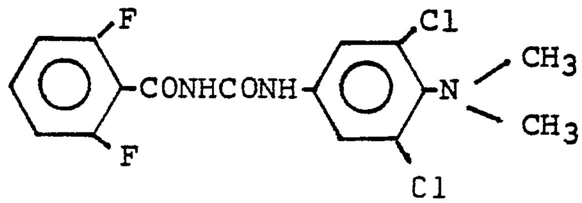 aus EP 16 729

II 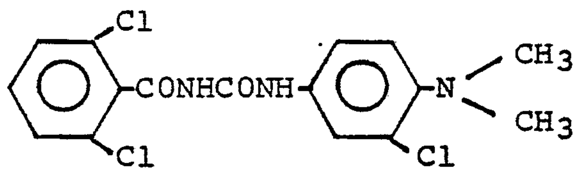 aus J.Agr.Food Chem. 21, 353 (1973)

Zuchtversuch mit Maiszünsler-Larven (Ostrinia nubilalis)

Die Zucht erfolgt auf einem Nährboden folgender Zusammensetzung:

515 g Maismehl

130 g Weizenkeime

137 g Bierhefe

18 g Ascorbinsäure

10 g Cellulosepulver

5 g Nipagin

20 g Wessons Salz

20 ml Vitaminlösung

80 g Agar

3100 ml Wasser

Im Ersttest, der mit einer End-Konzentration von 20 ppm Wirkstoff im Nährboden durchgeführt wird, verwendet man 50 ml Substrat in 100 ml Plastikbechern und 5 Raupen (L3).

Alle weiteren Versuchsstufen laufen doppelt in 250 ml Bechern mit 100 ml Nährsubstrat und 10 Raupen (d.h. 20 Raupen pro Konzentration). Es ist darauf zu achten, daß die Wirkstoffaufbereitung sorgfältig untergemischt wird, um eine gleichmäßige Verteilung zu gewährleisten. Die Beobachtung erstreckt sich bis zum Schlupf der Falter.

Als Vergleichsmittel dient Diflubenzuron.

Bei diesem Versuch erzielte die Verbindung des Beispiels 1 bei 0,4 ppm und die des Beispiels 19 bei 10,0 ppm 100 %ige Mortalität, während Vergleichsmittel I und II bei 4,0 bzw. 40,0 ppm 80 % bzw. 40 % Mortalität erzielten.

Zuchtversuch mit Baumwollwanzen (Dysdercus intermedius)

Baumwollwanzen Dysdercus intermedius werden im 4. Larvenstatium in Petrischalen (Ø 10 cm) dem Wirkstoffbelag der Testsubstanz für 24 Stunden ausgesetzt.

Die Überlebenden züchtet man in 1 l-Gläsern auf feuchtem Quarzsand, der vorher mit der Wirkstoffauflösung versetzt wurde bis zum Schlüpfen der $F_1$-Generation.

Dabei entsprechen in der Behandlung

2,5 mg/Schale 25 ppm im Sand

1,0 mg/Schale 10 ppm im Sand

0,5 mg/Schale 5 ppm im Sand

usw.

Beurteilt wird Mortalität und Vermehrung.

Bei diesem Versuch bewirkten die Verbindungen der Beispiele 1, 19, 43, 10, 5 und 41 bei einer wesentlich geringeren Konzentration als Vergleichsmittel I und II vollständige Unterdrückung der $F_1$-Generation.

Zuchtversuch mit Moskito-Larven (Aedes aegypti)

200 ml Leitungswasser werden mit der Wirkstoffaufbereitung versetzt und darauf mit 20 -30 Moskitolarven im 3. -4. -Larvenstadium besetzt.

Die Gefäße stehen bei 25°C. Beobachtet werden Verpuppung und Schlüpfen der Imagines, welches nach 10 -12 Tagen erfolgt.

Während der Beobachtungszeit wird einmal mit gepulvertem Zierfischfutter gefüttert.

Bei diesem Versuch erzielten die Verbindungen der Beispiele 1, 19, 43, 10, 5 und 41 Mortalität bei durchschnittlich 0,2 ppm, während Vergleichsmittel II bei 1,0 ppm nur 60 % Mortalität erzielte.

Entwicklungshemmung bei Prodenia litura

Prüfung auf behandeltem Nährboden)

100 g des Standard-Nährbodens für Prodenia werden in 250 ml-Becher gefüllt und warm mit der wäßrigen Wirkstoffaufbereitung sorgfältig gemischt.

Nach Erkalten belegt man jedes Gefäß mit 10 1-3-Raupen (1,5 -1,8 cm) und lagert sie bei 23°C. Beurteilt wird Verpuppung und Schlupf der Falter.

Pro Konzentration müssen mindestens 2 Becher angesetzt werden.

Bei diesem Versuch erzielten die Beispiele 1, 43, 10, 5 und 41 100 % Mortalität bei einer Konzentration von 0.2 ppm oder weniger.

**Ansprüche**

1. N-Benzoyl, N'-(aminophenyl)harnstoffe der Formel

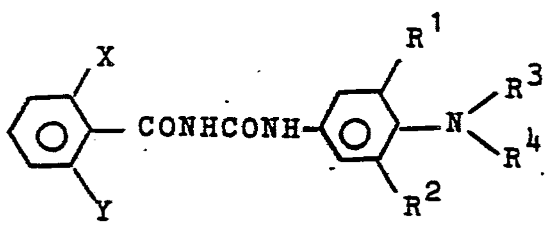

(I),

in der

X für F, Cl oder H,

Y für F, Cl,

$R^1$ und $R^2$ (die gleich oder verschieden sein können) für Cl, Br,

$R^3$ für einen gesättigten, olefinisch oder acetylenisch ungesättigten Kohlenwasserstoffrest mit 1 bis 5 Kohlenstoffatomen steht, der einen Phenylrest trägt, und $R^4$ für H oder einen acyclischen Kohlenwasserstoffrest mit 1 bis 5 Kohlenstoffatomen oder die Bedeutung von $R^3$ steht, wobei $R^3$ und $R^4$ gleich oder verschieden sein können.

2. Insektizides und akarizides Mittel, enthaltend einen N-Benzoylharnstoff der Formel I gemäß Anspruch 1.

3. Insektizides und akarizides Mittel, enthaltend übliche Formulierungshilfsmittel und einen N-Benzoyl-N'-phenylharnstoff der Formel I gemäß Anspruch 1.

4. Verfahren zur Bekämpfung von Insekten und Akariden, dadurch gekennzeichnet, daß man eine wirksame Menge eines N-Benzoyl-N'-phenylharnstoffs der Formel I gemäß Anspruch 1 auf die Schädlinge und/oder deren Lebensraum einwirken läßt.

5. Verfahren zur Herstellung eines insektiziden und akariziden Mittels, dadurch gekennzeichnet, daß man mindestens einen N-Benzoyl-N'-phenylharnstoff der Formel I gemäß Anspruch 1 mit oberflächenaktiven Verbindungen und/oder Streckmitteln und/oder weiteren Wirkstoffen mischt.

6. Verfahren zur Herstellung von N-Benzoyl-N'-phenylharnstoffen der Formel I gemäß Anspruch 1, dadurch gekennzeichnet, daß man entweder ein entsprechendes Benzoylisocyanat der Formel II

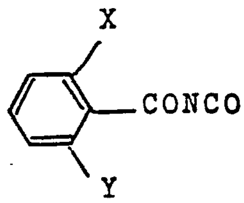

(II),

mit einem entsprechenden Aminoanilin der Formel III

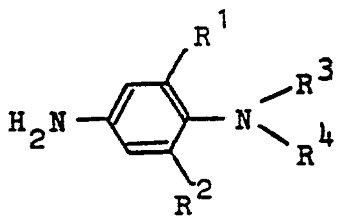

(III),

oder ein entsprechendes Amid der Formel IV

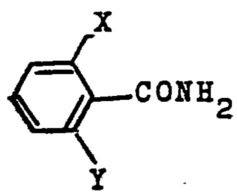

(IV),

mit einem entsprechenden Isocyanat der Formel IV

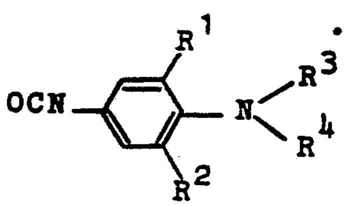

(V),

umsetzt.

Europäisches Patentamt

# EUROPÄISCHER RECHERCHENBERICHT

Nummer der Anmeldung

EP 86108020.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | <u>DE - A1 - 3 132 020</u> (BASF)<br>* Patentansprüche 1,4-10; Seiten 18, 19, Verbindungen 93-96 *<br>-- | 1-6 | C 07 C 127/22<br>A 01 N 47/34 |
| A | <u>DE - A1 - 3 235 419</u> (CIBA-GEIGY)<br>* Patentansprüche 1,13-15 *<br>-- | 1,2,4,6 | |
| A | <u>DE - A1 - 3 233 341</u> (CIBA-GEIGY)<br>* Patentansprüche 1,11-13 *<br>-- | 1,2,4,6 | |
| A | <u>DE - A1 - 3 233 383</u> (CIBA-GEIGY)<br>* Patentansprüche 1,11-13 *<br>-- | 1,2,4,6 | |
| A | <u>EP - A1 - 0 044 278</u> (CIBA-GEIGY)<br>* Patentansprüche 1,12-14 *<br>-- | 1,2,4,6 | RECHERCHIERTE SACHGEBIETE (Int. Cl.4) |
| D,A | <u>EP - A2 - 0 016 729</u> (CIBA-GEIGY)<br>* Patentansprüche 1,9-11 *<br>---- | 1,2,4,6 | C 07 C 127/00 |

Der vorliegende Recherchenbericht wurde für alle Patentansprüche erstellt.

| Recherchenort | Abschlußdatum der Recherche | Prüfer |
|---|---|---|
| WIEN | 11-09-1986 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument